# FASCICULE DE BREVET EUROPEEN

(11) **EP 4 178 702 B1**
(45) Date de publication et mention de la délivrance du brevet: **14.10.2026**
(21) Numéro de dépôt: 21746776.0
(22) Date de dépôt: 08.07.2021
(51) Int. Cl.: B01D 15/00, C07C 7/13, C07C 41/36

(54) **PURIFICATION DE LIQUIDES AROMATIQUES**
REINIGUNG VON AROMATISCHEN FLÜSSIGKEITEN
PURIFICATION OF AROMATIC LIQUIDS

(30) Priorité: 10.07.2020 FR 2007312
(43) Date de publication de la demande: 17.05.2023
(73) Titulaire: Eastman Chemical HTF GMBH, 45772 Marl (DE)
(72) Inventeur: BLANC, Jérôme, 69491 PIERRE-BENITE Cedex (FR); MONGUILLON, Bernard, 92705 COLOMBES CEDEX (FR); BOUVIER, Ludivine, 64170 LACQ (FR); RAVON, Ugo, 64170 LACQ (FR)
(74) Mandataire: Wallinger Ricker Schlotter PartGmbB
(86) Numéro de dépôt international: PCT/FR2021/051267
(87) Numéro de publication internationale: WO 2022/008846

(56) Documents cités:
- WO-A1-02/14240
- WO-A1-2013/180888
- CN-A- 104 230 619
- CN-A- 106 608 794
- US-A- 5 744 686
- US-A1- 2008 139 857
- US-B1- 10 093 597
- ASLAM RABYA ET AL: "Development of a liquid chromatographic method for the separation of a liquid organic hydrogen carrier mixture", SEPARATION AND PURIFICATION TECHNOLOGY, ELSEVIER SCIENCE, AMSTERDAM, NL, vol. 163, 24 February 2016 (2016-02-24), pages 140 - 144, XP029466427, ISSN: 1383-5866, DOI: 10.1016/J.SEPPUR.2016.01.051

## Description

La présente invention concerne le domaine de la purification des composés liquides, en particulier des composés liquides aromatiques, et plus particulièrement des composés liquides comportant au moins un, de préférence au moins deux, cycle(s) aromatique(s).

De nombreux domaines d'applications utilisent aujourd'hui des composés liquides aromatiques. Dans certains cas les composés liquides aromatiques sont soumis à diverses contraintes, et en particulier des contraintes thermiques plus ou moins importantes, pendant des durées plus ou moins importantes, et ce bien souvent avec des fréquences répétées.

Or les composés liquides aromatiques, lorsqu'ils sont soumis à des contraintes thermiques, et en particulier des contraintes thermiques importantes et répétées, peuvent avoir tendance à se dégrader, diminuant ainsi drastiquement la durée d'utilisation desdits composés liquides aromatiques, tout en générant des produits de dégradation qui peuvent au mieux diminuer le rendement, voire conduire à une diminution de la durée de vie du composé liquide aromatique dans l'application considérée et, selon un aspect plus ennuyeux voire dangereux, conduire à des produits de dégradation toxiques pour l'environnement, voire pour les êtres vivants.

En particulier, les dégradations des composés liquides aromatiques sont généralement observées au cours du temps à des températures plus ou moins proches de leur limite de stabilité. Les produits de dégradation sont le plus souvent classés en deux catégories : les sous-produits de dégradation à bas point d'ébullition et à bas point éclair dits « légers », d'une part, et les autres sous-produits de dégradation, dits « lourds » et qui généralement comportent un ou plusieurs cycles, éventuellement totalement ou partiellement insaturés que l'on peut qualifier dans la suite de produits « poly-aromatiques » et produits « polycycliques ».

Les sous-produits de dégradation à bas point d'ébullition et à bas point éclair peuvent entraîner des problèmes de stabilité et/ou de sécurité lors de l'utilisation, notamment des risques d'incendies, des problèmes de cavitation de pompes, ou encore des montées en pression dans les appareillages.

Ces sous-produits de dégradation à bas point d'ébullition et à bas point éclair peuvent le plus souvent être éliminés par soutirage de la phase vapeur présente dans lesdits composés liquides aromatiques, surtout lorsque ceux-ci sont portés à des températures plus élevées que les températures d'ébullition des produits de dégradation formés.

Ces produits de dégradation « légers » peuvent également être séparés par divers moyens basés sur les différences de propriétés physico-chimiques avec les composés liquides aromatiques d'intérêt, et notamment par décantation, cristallisation, recristallisation, et autres, ainsi que les combinaisons de deux ou plusieurs de ces méthodes. De tels moyens restent cependant très consommateurs de temps et d'énergie, ce qui les rend incompatibles avec des applications industrielles rentables.

Les autres produits de dégradation, dits « lourds » poly-aromatiques et polycycliques peuvent, de manière similaire, être séparés par divers moyens basés sur les différences de propriétés physico-chimiques avec les composés liquides aromatiques d'intérêt, et par exemple par cristallisation, recristallisation, et autres, ainsi que les combinaisons de deux ou plusieurs de ces méthodes. Comme indiqué précédemment, de tels moyens restent cependant très consommateurs de temps et d'énergie, ce qui les rend également incompatibles avec des applications industrielles rentables.

Un moyen pour pallier ces problèmes consiste généralement à remplacer le composé liquide aromatique usagé, c'est-à-dire souillé par les sous-produits de dégradation. Cette solution implique généralement la mise à l'arrêt des installations, la vidange du composé liquide aromatique comprenant les impuretés générées, ainsi que le traitement dudit composé liquide aromatique souillé par les impuretés. On comprend aisément qu'une telle solution représente une perte de temps, de rendement, et par conséquent un surcoût d'exploitation qui peut s'avérer important.

Bien souvent, les industriels traitent les sous-produits « légers » par soutirage de la phase vapeur, comme indiqué précédemment, tandis que les sous-produits « lourds » s'accumulent plus ou moins rapidement et impactent de manière très négative les rendements et/ou les performances des systèmes dans lesquels sont utilisés les composés liquides aromatiques.

Le document US 10,093,597 décrit un procédé de séparation d'isomères 3,3'-, 3,4'- et 4,4'-diméthyl biphényle, comprenant une mise en contact d'un mélange comprenant les isomères avec un adsorbant contenant une zéolithe.

Le document CN 104230619 décrit un procédé de purification d'hydrocarbures aromatiques contenant des sulfures et nitrures, comprenant une mise en contact avec un adsorbant comprenant de 0,01 à 70% d'un élément métal transition ou terre rare et de 30 à 99,99% d'un tamis moléculaire de type X.

Le document US 2008/0139857 décrit un procédé de purification de mélanges contenant un composé aromatique, comprenant une mise en contact avec une première zéolithe ayant une taille de pores moyenne de 0,3 à 0,5 nm et une deuxième zéolithe ayant une taille de pores moyenne de 0,6 à 0,8 nm.

Le document WO 2013/180888 décrit un procédé d'élimination de phénol dans un mélange comprenant des hydrocarbures aromatiques et en particulier du paraxylène, par mise en contact avec un adsorbant approprié.

Le document US 5,744,686 décrit un procédé d'élimination de composés azotés à partir d'un flux d'hydrocarbures aromatiques, par mise en contact avec un adsorbant sélectif ayant une taille de pores moyenne inférieure à 0,55 nm.

Le document CN 106608794 décrit un procédé d'élimination d'impuretés oxygénées dans un flux d'hydrocarbures aromatiques, de sorte à réduire la teneur en impuretés oxygénées à moins de 10 ppm en masse.

Le document WO 02/14240 décrit un procédé d'élimination de composés polaires à partir d'un mélange aromatique, comprenant une mise en contact du mélange avec un adsorbant sélectif comprenant un tamis moléculaire ayant des dimensions de pores et/ou de cavités de surface supérieures à 0,56 nm, et à une température inférieure ou égale à 130°C.

Il reste donc un besoin important pour des solutions permettant de limiter ou ralentir la formation et/ou l'accumulation d'impuretés notamment les sous-produits « lourds » générés lors de la dégradation de composés liquides organiques, afin d'allonger la durée de vie desdits composés liquides aromatiques dans leurs utilisations, et ainsi éviter le rejet dans l'environnement de composés toxiques, etc., et notamment dans les utilisations où lesdits composés liquides aromatiques sont soumis à des contraintes thermiques plus ou moins importantes et répétées.

Il a maintenant été découvert de manière surprenante que les objectifs précités peuvent être résolus en totalité ou au moins en partie grâce à la présente invention. D'autres objectifs encore pourront apparaître dans la description de la présente invention qui suit.

En effet, les inventeurs ont maintenant découvert que la durée de vie de composés liquides aromatiques peut être grandement améliorée en piégeant les produits de dégradation formés au cours de l'utilisation desdits composés liquides aromatiques, le piégeage étant réalisé par adsorption sélective desdits produits de dégradation.

Ainsi, et selon un premier aspect, l'invention concerne un procédé comprenant au moins une étape dans laquelle ledit composé liquide aromatique choisi parmi le benzyltoluène (BT), le dibenzyltoluène (DBT) et leurs mélanges, est mis en contact avec un matériau adsorbant zéolithique.

Par « composé liquide aromatique » on entend un composé comportant au moins un cycle aromatique et de préférence au moins deux cycles aromatiques, par exemple 2, 3 ou 4 cycles aromatiques, ainsi que leurs homologues totalement ou partiellement hydrogénés. Par homologues totalement ou partiellement hydrogénés, on entend que un cycle aromatique (ou plusieurs cycles aromatiques), est (ou sont) partiellement ou totalement hydrogéné(s). Les composés liquides aromatiques sont définis, sauf indication contraire, dans leurs formes totalement déshydrogénées, ceci signifiant que la définition comprend également lesdits composés liquides organiques dans leurs formes partiellement ou totalement hydrogénées. Parmi ces formes totalement ou partiellement hydrogénées, on préfère les composés liquides aromatiques dans lesquels il existe au moins un cycle aromatique dans sa forme totalement déshydrogénée.

Il a en effet été découvert de manière tout à fait surprenante que le traitement d'un composé liquide aromatique, éventuellement au moins partiellement ou totalement hydrogéné, peut être purifié, et en particulier la teneur en produits de dégradation peut être diminuée de manière importante, voire totale en mettant en contact ledit composé liquide avec un matériau adsorbant zéolithique autrement dit un matériau comprenant au moins un adsorbant présentant une ou plusieurs zéolithe(s), sous toute forme, en particulier sous forme de cristaux et/ou sous forme d'agglomérés zéolithiques.

Les produits de dégradation des composés liquides aromatiques susceptibles d'être éliminés, ou tout au moins dont la teneur peut être grandement diminuée, grâce au procédé ci-dessus sont généralement et le plus souvent les produits de dégradation les plus communément rencontrés, et parmi lesquels on peut citer, à titre d'exemples non limitatifs, le benzène, le toluène, le diméthylbenzène, l'éthyltoluène, l'aniline, le phénol, le naphtalène, ainsi que leurs formes partiellement ou totalement hydrogénées, telles que cyclohexane, méthyl-cyclohexane, et autres, et plus généralement encore les produits de dégradation apolaires aprotiques aromatiques, ou sous formes partiellement ou totalement hydrogénées, desdits composés liquides aromatiques.

La teneur en produits de dégradation des composés liquides aromatiques susceptibles d'être éliminés, ou tout au moins dont la teneur peut être grandement diminuée, peut varier dans de grandes proportions et est généralement comprise entre 1 ppm et 10000 ppm (massique).

Le composé liquide aromatique mis en oeuvre dans le procédé de purification peut être tout type de composé liquide à température et pression ambiantes (25°C, 1 atmosphère), comportant au moins un noyau aromatique, dans sa forme non hydrogénée, et de préférence au moins deux noyaux aromatiques, dans sa forme non hydrogénée, ledit composé liquide aromatique étant choisi parmi le benzyltoluène (BT), le dibenzyltoluène (DBT) et leurs mélanges. Le composé liquide aromatique utilisable dans le cadre du procédé peut éventuellement se présenter sous forme au moins partiellement, voire totalement, hydrogénée. Ces composés liquides aromatiques, éventuellement sous forme au moins partiellement, voire totalement, hydrogénée, sont généralement issus de produits pétroliers et/ou de produits synthétisés à partir de produits pétroliers, mais peuvent également être issus de produits renouvelables et/ou de produits synthétisés à partir de produits renouvelables.

Il doit être compris que le composé liquide aromatique mis en oeuvre dans le procédé peut se présenter sous forme de mélange de un ou plusieurs composés liquides aromatiques choisi parmi le benzyltoluène (BT), le dibenzyltoluène (DBT) et leurs mélanges, éventuellement partiellement ou voire totalement hydrogénés, et par exemple les mélanges de composés liquides aromatiques issus de produits pétroliers et/ou de produits renouvelables.

Par composés liquides aromatiques issus de produits pétroliers, on entend les produits issus de la séparation et/ou de la purification du pétrole, mais aussi les composés issus de la synthèse de composés porteurs de noyau(x) aromatique(s) d'origine pétrolière. Par composés liquides aromatiques issus de produits renouvelables, on entend les produits issus de la biomasse, et en particulier issus de l'extraction du bois (par exemple lignine) et de produits résineux, ainsi que les composés issus de synthèses de produits renouvelables.

Le composé liquide aromatique peut répondre à la formule générale (1) : ${\left(A-X\right)}_{n} - B$ (1) dans laquelle :
- A et B, identiques ou différents, représentent indépendamment l'un de l'autre, un cycle aromatique, éventuellement totalement ou partiellement hydrogéné, comportant éventuellement au moins, et de préférence, un hétéroatome, et éventuellement substitué par un ou plusieurs radicaux hydrocarbonés, saturés ou partiellement ou totalement insaturés, comportant de 1 à 20 atomes de carbone, de préférence de 1 à 18 atomes de carbone, de préférence encore de 1 à 12 atomes de carbone, mieux de 1 à 10 atomes de carbone, mieux encore de 1 à 6 atomes de carbone, typiquement de 1 à 3 atomes de carbone,
- X représente un groupement espaceur, choisi parmi une liaison simple, un atome d'oxygène, un atome de soufre, le radical bivalent (CRR')ₘ-, le radical bivalent >C=CRR', et le radical bivalent -NR"-, ou bien
   lorsque n est différent de 0 (zéro), X forme, avec les noyaux aromatiques auxquels il est rattaché, un cycle saturé ou insaturé comprenant de 4 à 10 sommets, parmi lesquels un ou plusieurs d'entre eux peut être un hétéroatome choisi parmi oxygène, azote, soufre, ledit cycle saturé ou insaturé pouvant en outre être substitué par une ou plusieurs chaînes hydrocarbonées comportant de 1 à 30 atomes de carbone, de préférence de 1 à 10 atomes de carbone,
- R et R', identiques ou différents, sont choisis indépendamment l'un de l'autre, parmi l'hydrogène et un radical hydrocarboné, saturé ou partiellement ou totalement insaturé, comportant de 1 à 6 atomes de carbone, de préférence de 1 à 3 atomes de carbone,
- R" représente un radical hydrocarboné, saturé ou partiellement ou totalement insaturé, comportant de 1 à 6 atomes de carbone, de préférence de 1 à 3 atomes de carbone,
- m représente un entier compris entre 1 et 4 bornes incluses, et
- n peut être égal à 0 ou représente un entier égal à 1, 2 ou 3, de préférence égal à 1 ou 2, avec la restriction que lorsque n est égal à 0, B est substitué par un ou plusieurs radicaux hydrocarbonés, comme défini précédemment.

Par « cycle aromatique », on entend les mono-cycles hydrocarbonés aromatiques et les polycycles hydrocarbonés aromatiques, comprenant de 6 à 20 atomes de carbone, parmi lesquels un ou plusieurs d'entre eux peuvent être des hétéroatomes choisis parmi oxygène, soufre et azote, de préférence parmi soufre et azote, et de préférence encore azote. Par « polycycle », on entend les cycles définis ci-dessus, fusionnés ou condensés, par exemples deux, ou plus de préférence deux ou trois ou quatre, de préférence encore deux ou trois, par exemple deux, cycles fusionnés ou condensés.

Lorsque n est égal à 0, le composé liquide aromatique de formule (1) définie ci-dessus fait partie de la famille des alkylbenzènes, éventuellement partiellement ou totalement hydrogénés. Lorsque n est égal à 2 ou 3, les groupements (A-X) peuvent être identiques ou différents.

Dans le composé liquide aromatique de formule générale (1), n peut être égal à 0 et le liquide organique de formule (1) peut être généralement choisi parmi les alkylbenzènes linéaires, éventuellement totalement ou partiellement hydrogénés, et les alkylbenzènes ramifiés, éventuellement totalement ou partiellement hydrogénés, tels que par exemple et de manière non limitative les alkylbenzènes, et homologues totalement ou partiellement hydrogénés, dans lesquels la partie alkyle comprend de 10 à 20 atomes de carbone. De tels alkylbenzènes comprennent, toujours de manière non limitative, le décylbenzène, le dodécylbenzène, l'octadécylbenzène, ainsi que leurs homologues totalement ou partiellement hydrogénés, pour ne citer que quelques-uns d'entre eux.

Le composé liquide aromatique de formule générale (1) peut présenter au moins deux cycles aromatiques, et dans ce cas n est différent de 0 et B est substitué par un radical hydrocarboné. De préférence encore ledit radical hydrocarboné est un radical alkyle comprenant de 1 à 6 atomes de carbone, de préférence de 1 à 4 atomes de carbone et de préférence le radical alkyle est le radical méthyle.

Comme indiqué précédemment, le composé liquide aromatique répondant à la formule générale (1) ci-dessus peut être utilisé seul ou en mélange de deux ou plusieurs d'entre eux en toutes proportions. Le composé liquide aromatique mis en œuvre dans le procédé peut contenir un composé porteur d'au moins un radical aromatique, éventuellement partiellement ou totalement hydrogéné, ou un mélange de deux ou plusieurs composés porteurs d'au moins un radical aromatique, éventuellement partiellement ou totalement hydrogéné, ledit composé liquide aromatique étant choisi parmi le benzyltoluène (BT), le dibenzyltoluène (DBT) et leurs mélanges,. Comme indiqué précédemment, le composé liquide aromatique mis en œuvre dans le procédé est liquide à température ambiante et pression ambiantes.

Selon l'invention, le composé liquide aromatique est choisi parmi le benzyltoluène (BT), le dibenzyltoluène (DBT), ainsi que leurs mélanges en toutes proportions.

Le composé liquide aromatique peut être choisi parmi les fluide organiques vendus par la société Arkema sous les dénominations commerciales de la gamme Jarytherm^{®}.

D'autres composés liquides aromatiques, et homologues partiellement ou totalement hydrogénés sont par exemple ceux commercialisés par la société Eastman, et notamment sous la dénomination commerciale Marlotherm^{®}.

Comme autres exemples encore de composés liquides aromatiques , on peut citer :
- le diphényléthane (DPE) et ses isomères, en particulier le 1,1-DPE (CAS 612-00-0), le 1,2-DPE (CAS 103-29-7) et leurs mélanges (notamment CAS 38888-98-1), de tels liquides organiques étant disponibles commercialement ou décrits dans la littérature, par exemple dans le document EP0098677,
- le ditolyléther (DT) et ses isomères, en particulier ceux répondant aux numéros CAS 4731-34-4, CAS 28299-41-4 et leurs mélanges, ceux-ci étant notamment disponibles dans le commerce auprès de la société Lanxess, sous la dénomination Diphyl DT,
- le phénylxylyléthane (PXE) et ses isomères, en particulier ceux répondant aux numéros CAS 6196-95-8, CAS 76090-67-0 et leurs mélanges, notamment disponibles commercialement auprès de la société Changzhou Winschem, sous la dénomination commerciale PXE oil,
- les mono- et bi-xylylxylènes, leurs isomères et leurs mélanges (CAS 186466-85-3),
- le 1,2,3,4-tétrahydro-(1-phényléthyl)naphtalène (CAS 63674-30-6), ce produit étant commercialement disponible notamment chez Dow sous la référence Dowtherm^{™} RP,
- le di-isopropylnaphtalène (CAS 38640-62-9), notamment disponible auprès de la société Indus Chemie Ltd, sous la dénomination commerciale KMC 113,
- le mono-isopropylbiphényle et ses isomères (CAS 25640-78-2), notamment disponible sous la dénomination commerciale Wemcol,
- le phényléthylphényléthane (PEPE) et ses isomères (CAS 6196-94-7), notamment disponible auprès de la société Changzhou Winschem ou de la société Yantaï Jinzheng,
- le N-éthylcarbazole, notamment disponible auprès de Allessa GmbH,
- les phénylpyridines, les tolylpyridines, les diphénylpyridines, les dipyridylbenzènes, les dipyridinetoluènes,
- ainsi que leurs homologues totalement ou partiellement hydrogénés,
- et les mélanges de deux ou plusieurs d'entre eux, en toutes proportions,
pour ne citer que les principaux liquides organiques connus et utilisables.

Comme indiqué précédemment le procédé permet de purifier un composé liquide organique, par mise en contact dudit composé liquide avec un matériau adsorbant zéolithique. Les matériaux adsorbants zéolithiques, c'est-à-dire les matériaux comprenant une ou plusieurs zéolithes, sont bien connus de l'homme du métier pour éliminer de petites molécule, généralement présentes à l'état de traces, des flux gazeux ou liquides.

En outre, les matériaux adsorbants zéolithiques comprennent le plus souvent des zéolithes synthétiques qui offrent, de par leur grande variété de procédés de préparation, une grande diversité de paramètres qui peuvent être finement ajustés, tels que par exemple la stabilité thermique, la résistance mécanique ou encore la facilitation de régénération, et ceci afin de répondre aux critères spécifiques requis pour l'utilisation d'intérêt.

Les matériaux adsorbants zéolithiques qui peuvent être utilisés dans le cadre de la présente invention peuvent être de tous types bien connus de l'homme du métier. Parmi les matériaux adsorbants zéolithiques les plus adaptés, on peut citer les zéolithes naturelles ou synthétiques, et plus particulièrement les matériaux adsorbants zéolithiques choisis parmi les zéolithes naturelles, comme par exemple la chabazite, et parmi les zéolithes de type LTA, les zéolithes de type FAU, les zéolithes de type EMT, les zéolithes de type MFI, et les zéolithes de type *BEA. Ces différents types de zéolithes sont aisément accessibles à l'homme du métier dans le commerce ou facilement synthétisables à partir de modes opératoires connus et disponibles dans la littérature scientifique et la littérature brevet. En outre les divers types de zéolithe sont clairement définis et exposés par exemple dans « Atlas of Zeolite Framework Types », 5e édition, (2001), Elsevier.

Pour les besoins de la présente invention, il est possible d'utiliser, en tant que matériaux adsorbants zéolithiques, des mélanges de deux ou plusieurs zéolithes, en toutes proportions. Il est également possible d'utiliser les homologues à porosité hiérarchisée des zéolithes précitées (dites « ZPH ») qui sont généralement obtenues par synthèse directe, notamment à l'aide d'agents sacrificiels, comme décrit par exemple dans les demandes WO2015019013 ou WO2007043731, ou bien par post-traitement de surface, comme décrit par exemple dans WO2013106816.

Les zéolithes listées ci-dessus peuvent être utilisées sous leur forme « native », c'est-à-dire sous la forme de cristaux, mais sont de préférence utilisées sous forme d'agglomérés de cristaux de zéolithes avec un ou plusieurs liants, selon des techniques bien connues de l'homme du métier, et notamment par agglomération de cristaux de zéolithes avec un liant d'agglomération. Le liant d'agglomération peut être de tout type permettant l'agglomération et la cohésion des cristaux de zéolithes et est généralement choisi parmi les argiles minérales, parmi lesquelles on peut citer, à titre d'exemples non limitatifs le kaolin, la kaolinite, l'attapulgite, la sépiolite, la clinoptilolite, et autres ainsi que les mélanges de deux ou plusieurs de ces argiles, en toutes proportions.

Les cristaux de zéolithes sont ainsi avantageusement agglomérés avec au moins un liant d'agglomération, et, si nécessaire ou si désiré, un ou plusieurs additifs bien connus de l'homme du métier, avant d'être séchés et/ou cuits et/ou calcinés.

Les additifs sont également bien connus de l'homme du métier et leur nature et quantité ajoutée peuvent varier dans de grandes proportions selon l'effet recherché ou requis. Des exemples d'additifs qui peuvent être mis en oeuvre avec les liants d'agglomération comprennent, à titre non limitatif, les additifs de passivation de surface qui ont pour but de gérer la réactivité de surface des agglomérés et/ou en améliorer la sélectivité de séparation, par exemple le pyrophosphate de tétrasodium (TSPP), les additifs rhéologiques, les additifs de granulation, et autres, ainsi que les mélanges de deux ou plusieurs d'entre eux.

Les cristaux de zéolithe agglomérés peuvent également être engagés dans une opération de zéolithisation, également bien connue de l'homme du métier, consistant à transformer tout ou partie du liant d'agglomération en matériau cristallin zéolithique, afin d'augmenter les capacités d'adsorption desdits agglomérés. Les techniques d'agglomération de cristaux de zéolithe, de séchage, cuisson, calcination et zéolithisation, sont parfaitement décrits dans la littérature scientifique et la littérature brevets et par exemple dans les demandes WO1999010096 et WO2000050166.

Les zéolithes (cristaux et agglomérés) indiquées ci-dessus comportent généralement et le plus souvent des cations afin d'assurer leur neutralité électronique. Les cations les plus communément utilisés sont choisis parmi, à titre d'exemples non limitatifs, parmi les métaux alcalins, les métaux alcalino-terreux et les métaux de transitions, et plus particulièrement parmi les cations de sodium, de potassium, de calcium, de baryum, de strontium, de magnésium, de fer, de cuivre, et d'argent. Les matériaux adsorbants zéolithiques utilisables dans le cadre de la présente invention peuvent bien entendu contenir un ou plusieurs des cations listés précédemment.

La présence de cations dans les matériaux adsorbants zéolithiques utilisables dans le cadre de la présente invention résulte soit directement de la synthèse desdits matériaux adsorbants, notamment le cation sodium pour les zéolithes préparées à partir de solutions sodiques, soit par une ou plusieurs opérations d'échanges cationiques, selon des techniques classiques et bien connues de l'homme du métier, lesdits échanges pouvant être réalisés sur les cristaux de zéolithe initiale et/ou sur les agglomérés de cristaux de zéolithes, avant et/ou pendant et/ou après leur mise en forme, de préférence avant et/ou après leur mise en forme.

Le matériau adsorbant zéolithique utilisable dans le cadre de la présente invention peut en effet, si nécessaire ou si souhaité, et le plus souvent, être mis en forme, selon toute technique connue de l'homme du métier, et en particulier par extrusion, granulation, et autres, pour des mises en forme de type billes, filés, et autres, telles que par exemple les solides monolithiques et les membranes.

Selon un mode de réalisation du procédé de la présente invention, on préfère utiliser les matériaux adsorbants zéolithiques comprenant une ou plusieurs zéolithes choisies parmi :
- les zéolithes LTA, de manière préférée les zéolithes 5A, en particulier celles comprenant des cations de calcium, ainsi que leurs homologues à porosité hiérarchisée (zéolithes homologues comprenant mésopores et micropores)
- les zéolithes de type FAU, et en particulier les zéolithes LSX, MSX, X et Y, et plus particulièrement les zéolithes présentant un ratio atomique Si/Al compris entre 1 et 3, ainsi que leurs homologues à porosité hiérarchisée (zéolithes homologues comprenant mésopores et micropores), comme par exemple décrits dans les demandes WO2015019013, WO2015019014, WO2015028740, et WO2015028741,
- les zéolithes de type FAU, et en particulier les zéolithes présentant un ratio atomique Si/Al strictement supérieur à 3, et par exemple les zéolithes USY et les zéolithes Y désaluminées,
- les zéolithes EMT ou des phases zéolithiques d'inter-croissance EMT-FAU, présentant un ratio atomique Si/Al compris entre 1 et 4, ainsi que leurs homologues à porosité hiérarchisée (zéolithes homologues comprenant mésopores et micropores), comme par exemple décrits dans la demande WO2014177567A1,
- le zéolithes de type MFI, typiquement les zéolithes présentant un ratio atomique Si/Al compris entre 8 et 500, de préférence entre 8 et 250, de préférence encore entre 8 et 100, avantageusement entre 8 et 50, mieux encore entre 8 et 40, et tout particulièrement les zéolithes ZSM-5, ainsi que leurs homologues à porosité hiérarchisée (zéolithes homologues comprenant mésopores et micropores), et
- les zéolithes de type *BEA, typiquement les zéolithes BETA présentant un ratio atomique Si/Al supérieur à 7, et de préférence un ratio atomique Si/Al compris entre 8 et 20.

Dans un mode de réalisation préféré, un matériau adsorbant zéolithique tout particulièrement adapté pour les besoins du procédé selon la présente invention est un matériau comprenant une zéolithe de type FAU, comportant un ou plusieurs cations choisis parmi Na, K, Ba, Ca, Mg, Li, Sr, Ag, Cu, et plus particulièrement NaX, BaX, BaKX, NaCaX, CaBaNaX, NaY, BaY, NaKY, BaKY, et leurs mélanges. Ces zéolithes sont disponibles dans le commerce et la plupart d'entre elles sont commercialisées par la société Arkema.

Le procédé de purification d'un composé liquide aromatique selon la présente invention comprend ainsi au moins une étape dans laquelle ledit composé liquide est mis en contact avec un matériau adsorbant zéolithique tel qu'il vient d'être défini. Il doit être compris que le procédé de la présente invention met en oeuvre un ou plusieurs matériaux adsorbants zéolithiques tels qu'ils viennent d'être définis.

Cette étape de mise en contact peut être avantageusement effectuée à une température comprise entre -20 °C et 250°C, de préférence entre -15 °C et 150°C, de préférence entre -10 °C et 100 °C, de préférence entre -5°C et 80°C, de préférence entre -5°C et 50°C, avantageusement à température ambiante, c'est-à-dire à la température de travail, et plus spécifiquement sans qu'il soit procédé à un apport de chaleur ou de froid, pour des raisons évidentes d'économie du procédé de l'invention.

De même, l'étape de mise en contact peut être effectuée sous pression, à pression atmosphérique, ou sous dépression, voire sous vide. On préfère cependant opérer à pression atmosphérique, ou sous pression pouvant aller jusqu'à 20 bar (2 MPa), de préférence 2 bar (200 kPa), et de manière tout particulièrement préférée, sous pression atmosphérique, c'est-à-dire à la pression de travail, et plus spécifiquement sans qu'il soit procédé à un apport de pression ou de dépression, hormis les différences de pression apportées par les appareillages tel que pompes, vannes et autres, pour des raisons évidentes d'économie du procédé de l'invention.

La durée de mise en contact peut varier dans de grandes proportions, notamment en fonction de la nature et de la quantité des impuretés à éliminer, de la nature et de la quantité du matériau adsorbant zéolithique utilisé, de la nature et de la quantité du liquide à purifier, et du type de système de mise en contact utilisé. En outre la durée de mise en contact varie en fonction de la température et de la pression appliquées.

La mise en contact avec le matériau adsorbant zéolithique peut être effectuée selon toute méthode bien connue de l'homme du métier, en continu ou en batch, et par exemple par passage, forcé (pompes) ou par gravité, du liquide au travers dudit matériau adsorbant zéolithique, tel que dans une colonne à remplissage, ou bien encore par simple contact dans un réacteur, tel qu'un réacteur muni ou non d'un système d'agitation, et autre.

Plus spécifiquement, l'étape du procédé de mise en contact du liquide aromatique à purifier avec au moins un matériau adsorbant zéolithique peut être mis en oeuvre dans divers procédés statiques (ou batch), dynamiques, semi-continus ou continus. Pour ces derniers, le flux à purifier traverse généralement un lit d'adsorbant sur lequel les polluants sont retenus sélectivement en fonction de critères spécifiques comme par exemple la nature du polluant (polarité, diamètre, encombrement stérique), du type de flux (gaz, liquide) et des conditions de mise en oeuvre (température, pression), et autres.

L'étape de mise en contact peut ainsi s'effectuer en une ou plusieurs fois, en batch et/ou en statique, dans les fûts de stockage, sous agitation ou non, en dynamique ou en continu. De préférence, cette étape de purification a lieu avant toute étape de stockage du liquide à traiter et de préférence en dynamique à travers un lit d'adsorbant, de préférence à travers un lit fixe d'adsorbant. Ainsi, et à titre d'exemples non limitatifs, l'étape de mise en contact du procédé de l'invention peut être effectuée en batch, et dans ce cas un mode de réalisation consiste à déposer un lit d'adsorbant en fond de container dans lequel le liquide aromatique à purifier est stocké, pendant une durée variable en fonction du degré de pollution et de la nature des polluants à éliminer. Cette durée peut en effet varier dans de grandes proportions, et est généralement comprise entre quelques minutes à quelques jours, par exemple entre 1 heure et 48 heures.

En alternative, l'étape de mise en contact peut être réalisée en continu, selon tout procédé dynamique connu, et pour lequel le liquide à purifier traverse un lit de matériau adsorbant zéolithique, dans les conditions de températures et de pressions indiquées plus haut. Le débit de passage en continu dudit liquide au travers dudit lit d'adsorbant peut varier dans de grandes proportions en fonction du degré de pollution et de la nature des polluants à éliminer, mais est généralement adapté pour permettre un temps de contact généralement compris entre quelques minutes à quelques jours, par exemple entre 1 heure et 48 heures. Le lit de matériau adsorbant zéolithique peut être de tout type bien connu de l'homme du métier et en particulier un lit fixe, lit fluidisé ou lit mobile (simulé ou non). On préfère utiliser, dans le cas de mise en contact en continu, mettre en oeuvre un lit fixe avec régénération du tamis ou fonctionnement en deux adsorbeurs, un premier travaillant en adsorption et un second travaillant en désorption/régénération.

Selon un mode de réalisation tout particulièrement avantageux de l'invention, le matériau adsorbant zéolithique peut en effet être désorbé et/ou régénéré, en batch ou de manière continu, selon les techniques classiques de désorption et régénération, et notamment par traitement thermique et/ou au moyen d'un ou plusieurs solvants de désorption.

Ainsi le procédé de la présente invention met en oeuvre au moins un matériau adsorbant zéolithique comme indiqué précédemment qui peut se présenter sous diverses forme, et notamment un lit d'adsorbant, par exemple un ou plusieurs types de zéolithe sous forme de mélange de cristaux ou d'agglomérés, ou encore plusieurs lits d'adsorbants identiques ou différents dans le même adsorbeur, un ou plusieurs adsorbeurs pouvant être mis en oeuvre, en série et/ou en parallèle, afin d'éliminer le plus sélectivement et le plus complètement possible les impuretés présentes dans les fluides aromatiques, et notamment les impuretés monocycliques, telles que par exemple le toluène, le benzène, le méthylcyclohexane, le diméthylbenzène, l'éthyltoluène, l'aniline, le phénol, le naphtalène, ainsi que leurs homologues totalement ou au moins partiellement hydrogénés.

Plus spécifiquement encore, le procédé de l'invention comprend au moins les étapes suivantes :
a) fourniture d'un liquide aromatique comprenant au moins une impureté,
b) mise en contact dudit liquide aromatique avec au moins un matériau adsorbant zéolithique,
c) récupération dudit liquide aromatique comprenant ladite au moins une impureté en une concentration en poids de moins de 50%, de préférence de moins de 40%, de préférence de moins de 30%, de préférence encore de moins de 20% en poids par rapport au taux d'impureté présente dans le liquide de l'étape a), et
d) éventuellement régénération et/ou désorption dudit au moins un matériau adsorbant zéolithique.

Le procédé décrit ci-dessus est tout particulièrement adapté pour la purification de liquides aromatiques comportant au moins un noyau aromatique, et de préférence au moins deux noyaux aromatiques, et pollués par une ou plusieurs impuretés définies précédemment comme sous-produits générés lors de la dégradation de composés liquides organiques, et notamment les impuretés monocycliques, telles que le toluène, le benzène, le méthylcyclohexane, le diméthylbenzène, l'éthyltoluène, l'aniline, le phénol, le naphtalène, ainsi que leurs homologues totalement ou au moins partiellement hydrogénés pour ne citer que les principales d'entre elles, sans caractère limitatif.

Ainsi, le procédé selon la présente invention peut être mis en œuvre dans un grand nombre de domaines d'application, et notamment les domaines d'applications dans lesquels un liquide aromatique est soumis à des conditions de dégradation, telles que par exemple des variations thermiques, qu'elles soient importantes ou non, cycliques ou non, des modifications chimiques, qu'elles soient réversibles ou non, et autres.

À titre d'exemples non limitatifs de tels domaines d'application, on peut, entre autres, citer les domaines dans lesquels des liquides aromatiques sont utilisés en tant que fluides caloporteurs, fluides diélectriques ou bien encore les domaines dans lesquels des liquides aromatiques sont utilisés comme transporteurs liquides organiques d'hydrogène (encore dénommés selon l'acronyme « LOHC » pour « Liquid Organic Hydrogen Carrier » en langue anglaise), comme par exemple décrit dans la demande WO2014082801.

Le procédé de l'invention est en effet tout particulièrement adapté à la purification de liquides caloporteurs ou de liquides LOHC, et notamment aux liquides aromatiques que sont le benzyltoluène et le dibenzyltoluène, seuls ou en mélange en toutes proportions. Selon un mode de réalisation particulièrement préféré, le procédé de l'invention concerne la purification de benzyltoluène ou de dibenzyltoluène, ou de leurs mélanges, par mise en contact avec un ou plusieurs adsorbants zéolithiques à base d'une ou plusieurs zéolithe(s) de type FAU, comme indiqué précédemment.

Comme indiqué précédemment, le procédé de l'invention peut être mis en œuvre en batch ou en continu, une fois ou plusieurs fois, selon les besoins rencontrés dans le domaine d'application concerné.

Ainsi, et par exemple dans le cas de l'utilisation comme LOHC, la purification du liquide organique peut être est effectuée une ou plusieurs fois, avant ou après une ou plusieurs des étapes du procédé, et par exemple avant une étape de déshydrogénation et/ou avant une étape d'hydrogénation.

Enfin, et selon un autre aspect, il est également décrit l'utilisation d'un matériau adsorbant zéolithique tel qu'il vient d'être défini pour la purification d'un composé liquide aromatique, tel que défini plus haut.

## Revendications

1. Procédé de purification d'un composé liquide aromatique choisi parmi le benzyltoluène (BT), le dibenzyltoluène (DBT) et leurs mélanges, ledit procédé comprenant au moins une étape dans laquelle ledit composé liquide aromatique est mis en contact avec un matériau adsorbant zéolithique.

2. Procédé selon la revendication 1, dans lequel le matériau adsorbant zéolithique est un matériau comprenant au moins un adsorbant présentant une ou plusieurs zéolithe(s), sous forme de cristaux et/ou sous forme d'agglomérés zéolithiques.

3. Procédé selon l'une quelconque des revendications précédentes, dans lequel ledit matériau adsorbant zéolithique est choisi parmi les zéolithes naturelles ou synthétiques, et de préférence parmi la chabazite, les zéolithes de type LTA, les zéolithes de type FAU, les zéolithes de type EMT, les zéolithes de type MFI, et les zéolithes de type *BEA.

4. Procédé selon l'une quelconque des revendications précédentes, dans lequel ledit matériau adsorbant zéolithique comporte au moins un cation choisi parmi les métaux alcalins, les métaux alcalino-terreux et les métaux de transitions, et plus particulièrement parmi les cations de sodium, de potassium, de calcium, de baryum, de strontium, de magnésium, de fer, de cuivre, et d'argent.

5. Procédé selon l'une quelconque des revendications précédentes, dans lequel ledit matériau adsorbant zéolithique comprend une ou plusieurs zéolithes choisies parmi :
- les zéolithes LTA, de manière préférée les zéolithes 5A, en particulier celles comprenant des cations de calcium, ainsi que leurs homologues à porosité hiérarchisée,
- les zéolithes de type FAU, choisies parmi les zéolithes LSX, MSX, X et Y, et présentant un ratio atomique Si/Al compris entre 1 et 3, ainsi que leurs homologues à porosité hiérarchisée,
- les zéolithes de type FAU, choisies parmi les zéolithes présentant un ratio atomique Si/Al strictement supérieur à 3,
- les zéolithes EMT ou des phases zéolithiques d'inter-croissance EMT-FAU, présentant un ratio atomique Si/Al compris entre 1 et **4,** ainsi que leurs homologues à porosité hiérarchisée,
- les zéolithes de type MFI, typiquement les zéolithes présentant un ratio atomique Si/Al compris entre 8 et 500, de préférence entre 8 et 250, de préférence encore entre 8 et 100, avantageusement entre 8 et 50, mieux encore entre 8 et 40, et tout particulièrement les zéolithes ZSM-5, ainsi que leurs homologues à porosité hiérarchisée, et
- les zéolithes de type *BEA, typiquement les zéolithes BETA présentant un ratio atomique Si/Al supérieur à 7, et de préférence un ratio atomique Si/Al compris entre 8 et 20.

6. Procédé selon l'une quelconque des revendications précédentes, dans lequel ledit matériau adsorbant zéolithique est un matériau comprenant une zéolithe de type FAU, comportant un ou plusieurs cations choisis parmi Na, K, Ba, Ca, Mg, Li, Sr, Ag, Cu, et plus particulièrement NaX, BaX, BaKX, NaCaX, CaBaNaX, NaY, BaY, NaKY, BaKY, et leurs mélanges.

7. Procédé selon l'une quelconque des revendications précédentes, comprenant au moins les étapes suivantes :
a) fourniture d'un liquide aromatique comprenant au moins une impureté,
b) mise en contact dudit liquide aromatique avec au moins un matériau adsorbant zéolithique,
c) récupération dudit liquide aromatique comprenant ladite au moins une impureté en une concentration en poids de moins de 50%, de préférence de moins de 40%, de préférence de moins de 30%, de préférence encore de moins de 20% en poids par rapport au taux d'impureté présente dans le liquide de l'étape a), et
d) éventuellement régénération et/ou désorption dudit au moins un matériau adsorbant zéolithique.

## Patentansprüche

1. Verfahren zur Reinigung einer aromatischen flüssigen Verbindung, die aus Benzyltoluol (BT), Dibenzyltoluol (DBT) und Mischungen davon ausgewählt ist, wobei das Verfahren mindestens einen Schritt umfasst, wobei die aromatische flüssige Verbindung mit einem zeolithischen Adsorptionsmittel in Kontakt gebracht wird.

2. Verfahren nach Anspruch 1, wobei das zeolithische Adsorptionsmaterial ein Material ist, das mindestens ein Adsorptionsmittel umfasst, welches einen oder mehrere Zeolithe in Form von Kristallen und/oder in Form von zeolithischen Agglomeraten umfasst.

3. Verfahren nach einem der vorhergehenden Ansprüche, wobei das zeolithische Adsorptionsmaterial aus natürlichen oder synthetischen Zeolithen, und vorzugsweise aus Chabazit, Zeolithen vom LTA-Typ, Zeolithen vom FAU-Typ, Zeolithen vom EMT-Typ, Zeolithen vom MFI-Typ und Zeolithen vom *BEA-Typ, ausgewählt ist.

4. Verfahren nach einem der vorhergehenden Ansprüche, wobei das zeolithische Adsorptionsmaterial mindestens ein Kation umfasst, das aus Alkalimetallen, Erdalkalimetallen und Übergangsmetallen, sowie insbesondere aus Natrium-, Kalium-, Calcium-, Barium-, Strontium-, Magnesium-, Eisen-, Kupfer- und Silberkationen, ausgewählt ist.

5. Verfahren nach einem der vorhergehenden Ansprüche, wobei das zeolithische Adsorptionsmaterial einen oder mehrere Zeolithe umfasst, die aus Folgendem ausgewählt sind:
- LTA-Zeolithen, vorzugsweise 5A-Zeolithen, insbesondere solchen, die Calcium-Kationen enthalten, sowie ihren Gegenstücken, die eine hierarchische Porosität aufweisen,
- Zeolithen vom FAU-Typ, die aus LSX-, MSX-, X- und Y-Zeolithen ausgewählt sind, mit einem Si/Al-Atomverhältnis zwischen 1 und 3 sowie ihren Gegenstücken, die eine hierarchische Porosität aufweisen,
- Zeolithen vom FAU-Typ, die aus Zeolithen mit einem Si/Al-Atomverhältnis von strikt größer als 3 ausgewählt sind,
- EMT-Zeolithen oder EMT-FAU-Zeolith-Verwachsungsphasen mit einem Si/Al-Atomverhältnis zwischen 1 und 4 sowie ihren Gegenstücken, die eine hierarchische Porosität aufweisen,
- Zeolithen vom MFI-Typ, typischerweise Zeolithen mit einem Si/Al-Atomverhältnis zwischen 8 und 500, vorzugsweise zwischen 8 und 250, noch bevorzugter zwischen 8 und 100, vorteilhafterweise zwischen 8 und 50, noch bevorzugter zwischen 8 und 40, und insbesondere ZSM-5-Zeolithen sowie ihren Gegenstücken, die eine hierarchische Porosität aufweisen, und
- Zeolithen vom *BEA-Typ, typischerweise BETA-Zeolithen mit einem Si/Al-Atomverhältnis von größer als 7 und vorzugsweise einem Si/Al-Atomverhältnis zwischen 8 und 20.

6. Verfahren nach einem der vorhergehenden Ansprüche, wobei das zeolithische Adsorptionsmaterial ein Material ist, das einen Zeolith vom FAU-Typ umfasst, der ein oder mehrere Kationen umfasst, die aus Na, K, Ba, Ca, Mg, Li, Sr, Ag, Cu und insbesondere NaX, BaX, BaKX, NaCaX, CaBaNaX, NaY, BaY, NaKY, BaKY und Mischungen davon ausgewählt sind.

7. Verfahren nach einem der vorhergehenden Ansprüche, umfassend mindestens die folgenden Schritte:
a) Bereitstellen einer aromatischen Flüssigkeit, die mindestens eine Verunreinigung umfasst,
b) Inkontaktbringen der aromatischen Flüssigkeit mit mindestens einem zeolithischen Adsorptionsmittel,
c) Rückgewinnen der aromatischen Flüssigkeit, welche die mindestens eine Verunreinigung in einer Gewichtskonzentration von weniger als 50 Gew.-%, vorzugsweise weniger als 40 Gew.-%, vorzugsweise weniger als 30 Gew.-%, besonders bevorzugt weniger als 20 Gew.-% relativ zu dem Verunreinigungsgrad in der Flüssigkeit aus Schritt a) umfasst, und
d) optionales Regenerieren und/oder Desorbieren des mindestens einen zeolithischen Adsorptionsmaterials.

## Claims

1. A method for purifying an aromatic liquid compound selected from benzyltoluene (BT), dibenzyltoluene (DBT) and mixtures thereof, said method comprising at least one step wherein said aromatic liquid compound is brought into contact with a zeolitic adsorbent material.

2. The method according to claim 1, wherein the zeolitic adsorbent material is a material comprising at least one adsorbent comprising one or more zeolites, in the form of crystals and/or in the form of zeolitic agglomerates.

3. The method according to any one of the preceding claims, wherein the said zeolitic adsorbent material is selected from natural or synthetic zeolites, and preferably from chabazite, LTA-type zeolites, FAU-type zeolites, EMT-type zeolites, MFI-type zeolites, and *BEA-type zeolites.

4. The method according to any one of the preceding claims, wherein the said zeolitic adsorbent material comprises at least one cation selected from alkali metals, alkaline earth metals and transition metals, and more particularly from sodium, potassium, calcium, barium, strontium, magnesium, iron, copper, and silver cations.

5. The method according to any one of the preceding claims, wherein said zeolitic adsorbent material comprises one or more zeolites selected from:
- LTA zeolites, preferably 5A zeolites, particularly those containing calcium cations, as well as their counterparts having hierarchical porosity,
- FAU-type zeolites, selected from LSX, MSX, X and Y zeolites, and with an Si/Al atomic ratio between 1 and 3, as well as their counterparts having hierarchical porosity,
- FAU-type zeolites, selected from zeolites with an Si/Al atomic ratio strictly greater than 3,
- EMT zeolites or EMT-FAU zeolitic intergrowth phases, with an Si/Al atomic ratio between 1 and 4, as well as their counterparts having hierarchical porosity,
- MFI-type zeolites, typically zeolites with an Si/Al atomic ratio between 8 and 500, preferably between 8 and 250, more preferably between 8 and 100, advantageously between 8 and 50, even more preferably between 8 and 40, and particularly ZSM-5 zeolites, as well as their counterparts having hierarchical porosity, and
- *BEA-type zeolites, typically BETA zeolites with an Si/Al atomic ratio greater than 7, and preferably an atomic Si/Al ratio between 8 and 20.

6. The method according to any one of the preceding claims, wherein the said zeolitic adsorbent material is a material comprising an FAU-type zeolite, comprising one or more cations selected from Na, K, Ba, Ca, Mg, Li, Sr, Ag, Cu, and more particularly NaX, BaX, BaKX, NaCaX, CaBaNaX, NaY, BaY, NaKY, BaKY, and mixtures thereof.

7. The method according to any one of the preceding claims, comprising at least the following steps:
a) providing an aromatic liquid comprising at least one impurity,
b) bringing said aromatic liquid into contact with at least one zeolitic adsorbent material,
c) recovering of said aromatic liquid comprising the said at least one impurity in a concentration by weight of less than 50%, preferably less than 40%, preferably less than 30%, more preferably less than 20% by weight relative to the impurity level present in the liquid of step a), and
d) optionally regenerating and/or desorpting of the said at least one zeolitic adsorbent material.
